# EUROPEAN PATENT APPLICATION

(11) **EP 4 726 048 A1**
(43) Date of publication of application: **15.04.2026**
(21) Application number: 24826252.9
(22) Date of filing: 19.06.2024
(51) Int. Cl.: C12P 13/10, C12N 15/77

(54) **MICROORGANISM PRODUCING L-CITRULLINE AND METHOD FOR PRODUCING L-CITRULLINE USING SAME**

(30) Priority: 20.06.2023 KR 20230079225
(71) Applicant: CJ Cheiljedang Corporation, Seoul 04560 (KR)
(72) Inventor: HAN, Seunghee, Seoul 04560 (KR); LEE, Ji Yeon, Seoul 04560 (KR); PARK, So-Yeon, Seoul 04560 (KR)
(74) Representative: Meissner Bolte Partnerschaft mbB
(86) International application number: PCT/KR2024/008480
(87) International publication number: WO 2024/262937

(57) **Abstract**

Provided are a method of producing L-citrulline, the method comprising the step of culturing, in a medium, a microorganism of the genus *Corynebacterium* having an L-citrulline-producing ability, in which the activity of a protein comprising an amino acid sequence of SEQ ID NO: 1 is weakened, as compared to its endogenous activity; a composition for producing L-citrulline, the composition comprising the microorganism, a culture of the microorganism, a fermentation product of the microorganism, or a combination of two or more thereof; and use of the microorganism in producing L-citrulline.

## Description

### [Technical Field]

The present disclosure relates to a method of producing L-citrulline, the method comprising the step of culturing, in a medium, a microorganism of the genus *Corynebacterium* having an L-citrulline-producing ability, in which the activity of a protein comprising an amino acid sequence of SEQ ID NO: 1 is weakened, as compared to its endogenous activity; a composition for producing L-citrulline, the composition comprising the microorganism, a culture of the microorganism, a fermentation product of the microorganism, or a combination of two or more thereof; and use of the microorganism in producing L-citrulline.

### [Background Art]

L-citrulline, which is a glutamate-derived L-amino acid, is industrially useful, because it has the functions of dilating blood vessels and promoting ammonia excretion in the body (K Takeda et al. 2013).

Microorganisms of the genus *Corynebacterium,* particularly, *Corynebacterium glutamicum* are Gram-positive microorganisms frequently used in the production of L-amino acids. For the production of L-amino acids, target material-specific approaches are mainly used, such as increasing the expression level of a gene encoding an enzyme mainly involved in the L-amino acid biosynthesis in a strain of the genus *Corynebacterium,* or deleting a gene unnecessary for the L-amino acid biosynthesis (US 9644009 B2). However, there is still a growing need for research on methods capable of efficiently producing L-amino acids.

Accordingly, research is still needed to effectively increase the L-citrulline-producing ability.

### [Disclosure]

### [Technical Problem]

An object of the present disclosure is to provide a microorganism producing L-citrulline and a method of producing L-citrulline using the same.

### [Technical Solution]

An aspect of the present disclosure provides a method of producing L-citrulline, the method comprising the step of culturing, in a medium, a microorganism of the genus *Corynebacterium* having an L-citrulline-producing ability, in which the activity of a protein comprising an amino acid sequence of SEQ ID NO: 1 is weakened, as compared to its endogenous activity.

In one specific embodiment, the protein comprising the amino acid sequence of SEQ ID NO: 1 may be encoded by a gene comprising a nucleotide sequence of SEQ ID NO: 2.

With regard to the microorganism according to any one of the aforementioned specific embodiments, the microorganism of the genus *Corynebacterium* may be *Corynebacterium glutamicum.*

In another specific embodiment, the method may further comprise the step of recovering L-citrulline from the cultured microorganism, a culture of the microorganism, a fermentation product of the microorganism, or the culture medium.

Another aspect of the present disclosure provides a composition for producing L-citrulline, the composition comprising a microorganism of the genus *Corynebacterium* having an L-citrulline-producing ability, in which the activity of a protein comprising an amino acid sequence of SEQ ID NO: 1 is weakened, as compared to its endogenous activity, a culture of the microorganism, a fermentation product of the microorganism, or a combination of two or more thereof.

### [Advantageous Effects]

A microorganism of the genus *Corynebacterium* having an L-citrulline-producing ability of the present disclosure, in which the activity of a protein comprising an amino acid sequence of SEQ ID NO: 1 is weakened, as compared to its endogenous activity, may produce L-citrulline in a high yield, thereby being usefully applied to industrial production of L-citrulline.

### [Detailed Description of Preferred Embodiments]

The present disclosure will be described in detail as follows. Meanwhile, each description and embodiment disclosed in this disclosure may also be applied to other descriptions and embodiments. That is, all combinations of various elements disclosed in this disclosure fall within the scope of the present disclosure. Further, the scope of the present disclosure is not limited by the specific description described below.

Further, those skilled in the art will recognize, or be able to ascertain using no more than routine experimentation, many equivalents to the specific embodiments of the disclosure described herein. Further, these equivalents should be interpreted to fall within the present disclosure.

As used in the specification and appended claims of the present disclosure, the singular forms ("a", "an", and "the") comprise plural referents unless the context clearly states otherwise. Unless the context indicates otherwise, singular terms shall comprise the plural and plural terms shall comprise the singular. As used in the specification and appended claims of the present disclosure, unless stated otherwise, the use of "or" may be used to comprise "and/or".

As used herein, the term "about" may be presented before a particular numerical value. The term "about" used herein comprises not only the exact number recited after the term, but also a range that is near or close to that number. Considering the context in which the number is presented, it may be determined whether any number is close to or near the particular number presented. In one example, the term "about" may refer to a range from -10% to +10% of a numerical value. In another example, the term "about" may refer to a range from -5% to +5% of a given numerical value, but is not limited thereto.

As used herein, the descriptions such as the terms "first, second, third,," "i), ii), iii),," or "(a), (b), (c), (d)" are used to distinguish similar constitutions, and these terms do not mean that the constitutions are performed continually or sequentially. For example, when the terms are used in reference to steps of a method, use, or assay, there may be no time interval between these steps, or they may be performed concurrently, or may be performed several seconds, several minutes, several hours, several days, or several months apart.

As used herein, the term "consisting essentially of" may mean that, in the case where the features of the object claimed herein are not substantially affected by the presence of an unspecified component, the unspecified component may be present.

As used herein, the term, "consisting of" means that the total ratio of specific component(s) is 100%. The components or features that are recited after the term "consisting of" may be essential or mandatory. In some embodiments, in addition to the components or features recited after the term "consisting of", any other components or non-essential components may be excluded.

As used herein, the term "comprising" means the presence of features, steps or components recited after the term, and does not exclude the presence or addition of one or more features, steps or components. The components or features recited after the term "comprising" herein may be essential or mandatory. However, in some embodiments, the term may further comprise any other or non-essential components or features.

An aspect of the present disclosure provides a method of producing L-citrulline, the method comprising the step of culturing, in a medium, a microorganism of the genus *Corynebacterium* having an L-citrulline-producing ability, in which the activity of a protein comprising an amino acid sequence of SEQ ID NO: 1 is weakened, as compared to its endogenous activity.

As used herein, the term "protein comprising the amino acid sequence of SEQ ID NO: 1" refers to a hypothetical protein that endogenously exists in the microorganisms of the genus *Corynebacterium,* of which functions are unknown.

Specifically, the protein comprising the amino acid sequence of SEQ ID NO: 1 of the present disclosure may be a protein having the activity of the protein comprising the amino acid sequence of SEQ ID NO: 1 which is encoded by a gene comprising a nucleotide sequence of SEQ ID NO: 2, but is not particularly limited to its type, as long as it has the activity corresponding to that of the protein comprising the amino acid sequence of SEQ ID NO: 1 while its activity in a microorganism of the genus *Corynebacterium* is weakened as compared to its endogenous activity, thereby improving the ability to produce L-citrulline. The protein comprising the amino acid sequence of SEQ ID NO: 1 which is encoded by the gene comprising the nucleotide sequence of SEQ ID NO: 2 is known in the art, and the amino acid and polynucleotide sequences of the protein comprising the amino acid sequence of SEQ ID NO: 1 may be obtained from known databases, for example, NCBI GenBank, etc., but are not limited thereto.

In the present disclosure, the protein comprising the amino acid sequence of SEQ ID NO: 1 may be a protein comprising the amino acid sequence of SEQ ID NO: 1, derived from a microorganism of the genus *Corynebacterium* (*Corynebacterium* sp.), specifically, *Corynebacterium glutamicum,* but is not limited thereto.

For example, the protein comprising the amino acid sequence of SEQ ID NO: 1 may comprise the amino acid sequence of SEQ ID NO: 1 or an amino acid sequence having 60% or more homology or identity thereto, but is not limited thereto, as long as it has the activity of the protein comprising the amino acid sequence of SEQ ID NO: 1. Specifically, the polypeptide having the activity of the protein comprising the amino acid sequence of SEQ ID NO: 1 may have or comprise the amino acid sequence of SEQ ID NO: 1 or an amino acid sequence having at least 60%, 70%, 80%, 85%, 90%, 95%, 96%, 97%, 98%, or 99% or more homology or identity to SEQ ID NO: 1, or may consist of the amino acid sequence, or may essentially consist of the amino acid sequence. For example, the protein comprising the amino acid sequence of SEQ ID NO: 1 may refer to a protein that endogenously exists in the microorganisms of the genus *Corynebacterium* or *Corynebacterium glutamicum,* but is not limited thereto.

With respect to amino acid sequences in the present disclosure, although it is described as a polypeptide or protein "comprising" an amino acid sequence described by a specific sequence number, a polypeptide "consisting of" an amino acid sequence described by a specific sequence number, or a polypeptide or protein "having" an amino acid sequence described by a specific sequence number, it is apparent that any protein having an amino acid sequence in which part of the sequence is deleted, modified, substituted, conservatively substituted or added may fall within the scope of the present disclosure as long as it has the identical or corresponding activity to that of the protein consisting of the amino acid sequence of the corresponding sequence number. For example, as long as a protein has the identical or corresponding activity to that of the modified protein, it does not exclude addition of a sequence that does not change the function of the protein before or after the amino acid sequence, a naturally occurring mutation, a silent mutation thereof, or conservative substitution, and it is apparent that even though the protein has such sequence additions or mutations, it falls within the scope of the present disclosure.

For example, it may be a case of having addition of a sequence which does not change the function of the variant polypeptide of the present disclosure at the N-terminus, C-terminus, and/or inside of the amino acid sequence, a naturally occurring mutation, a silent mutation, or a conservative substitution. For example, the polypeptide may be conjugated to an N-terminal signal (or leader) sequence that is cotranslationally or post-translationally involved in the protein transfer. Further, the polypeptide may be conjugated to another sequence or linker so as to enable identification, purification, or synthesis of the polypeptide.

As used herein, the term "conservative substitution" refers to substitution of an amino acid with another amino acid having similar structural and/or chemical properties. Such amino acid substitution may generally occur based on similarity of polarity, charge, solubility, hydrophobicity, hydrophilicity, and/or amphipathic nature of a residue. For example, positively charged (basic) amino acids comprise arginine, lysine, and histidine; negatively charged (acidic) amino acids comprise glutamic acid and aspartic acid; amino acids having a nonpolar side chain (nonpolar amino acids) comprise glycine, alanine, valine, leucine, isoleucine, methionine, phenylalanine, tryptophan, and proline; amino acids having a polar or hydrophilic side chain (polar amino acids) comprise serine, threonine, cysteine, tyrosine, asparagine, and glutamine. For another example, amino acids may be classified into amino acids with electrically charged side chains (electrically charged amino acids) such as arginine, lysine, histidine, glutamic acid, and aspartic acid, and amino acids with uncharged side chains (uncharged amino acids; also called neutral amino acids) such as glycine, alanine, valine, leucine, isoleucine, methionine, phenylalanine, tryptophan, proline, serine, threonine, cysteine, tyrosine, asparagine, and glutamine. For still another example, phenylalanine, tryptophan, and tyrosine may be classified as aromatic amino acids. For still another example, valine, leucine, and isoleucine may be classified as branched amino acids. For still another example, 20 types of amino acids are classified according to size, starting from the amino acid group with relatively small volume, the amino acids may be classified into five groups; glycine, alanine, serine; cysteine, proline, threonine, aspartic acid, asparagine; valine, histidine, glutamic acid, glutamine; isoleucine, leucine, methionine, lysine, arginine; and phenylalanine, tryptophan, tyrosine. However, they are not necessarily limited thereto. Usually, conservative substitution may hardly affect or not affect activity of polypeptides.

Further, the nucleotide sequence encoding the protein comprising the amino acid sequence of SEQ ID NO: 1 may be a nucleotide sequence encoding a protein that exhibits the activity of the protein comprising the amino acid sequence of SEQ ID NO: 1 while its activity in the microorganisms of the genus *Corynebacterium* is weakened, as compared to its endogenous activity, thereby improving the ability to produce L-citrulline. For example, the nucleotide sequence may be NCgl2383 gene which is a gene encoding a hypothetical protein that endogenously exists in the microorganisms of the genus *Corynebacterium,* of which functions are unknown, but is not limited thereto.

For example, the protein comprising the amino acid sequence of SEQ ID NO: 1 may be encoded by a polynucleotide having or comprising the nucleotide sequence of SEQ ID NO: 2 or a nucleotide sequence having 60% or more, 70% or more, 75% or more, 80% or more, 85% or more, 90% or more, 95% or more, 96% or more, 97% or more, 98% or more, and less than 100% homology or identity to the nucleotide sequence of SEQ ID NO: 2, or consisting of or essentially consisting of the nucleotide sequence, but is not limited thereto. Further, the nucleotide sequence of SEQ ID NO: 2 may be obtained from known databases, for example, NCBI GenBank, etc., but is not limited thereto.

In the present disclosure, the gene comprising the nucleotide sequence of SEQ ID NO: 2 may be used interchangeably with a polynucleotide comprising the nucleotide sequence of SEQ ID NO: 2, a gene or polynucleotide having the nucleotide sequence of SEQ ID NO: 2, a gene or polynucleotide consisting of the nucleotide sequence of SEQ ID NO: 2, or NCgl2383 gene.

For example, the NCgl2383 gene, which is a gene comprising the nucleotide sequence of SEQ ID NO: 2, may have two open reading frames (ORFs), for example, ORF 1 and ORF 2, but is not limited thereto. For example, ORF 1 of the NCgl2383 gene may encode a protein comprising an amino acid sequence of SEQ ID NO: 17, and ORF 2 of the NCgl2383 gene may encode a protein comprising an amino acid sequence of SEQ ID NO: 18, but is not limited thereto.

The polynucleotide of the present disclosure may undergo various modifications in the coding region without changing the amino acid sequence of the protein comprising the amino acid sequence of SEQ ID NO: 1 of the present disclosure due to codon degeneracy or in consideration of the codons preferred in an organism that is intended to express the protein comprising the amino acid sequence of SEQ ID NO: 1 of the present disclosure. Therefore, it is apparent that a polynucleotide which may be translated to the polypeptide consisting of the amino acid sequence of the protein comprising the amino acid sequence of SEQ ID NO: 1 of the present disclosure or a polypeptide having homology or identity thereto due to codon degeneracy may also be comprised in the polynucleotide of the present disclosure. For example, the polynucleotide of the present disclosure may be SEQ ID NO: 2 or a degenerated sequence thereof.

Further, the polynucleotide of the present disclosure may comprise a probe which may be prepared from a known gene sequence, for example, any sequence without limitation as long as it hybridizes with a sequence complementary to the entirety or a part of the polynucleotide sequence of the present disclosure under stringent conditions to encode the protein comprising the amino acid sequence of SEQ ID NO: 1 of the present disclosure.

As used herein, the term "homology" or "identity" refers to a degree of relevance between two given amino acid sequences or nucleotide sequences, and may be expressed as a percentage. The terms "homology and identity" may often be used interchangeably with each other.

The sequence homology or identity of a conserved polynucleotide or polypeptide is determined by standard alignment algorithms, and the default gap penalty established by a program to be used may be used together. Substantially, homologous or identical sequences may generally hybridize under moderately or highly stringent conditions to the entirety of the sequence or at least about 50%, 60%, 70%, 80%, or 90% of the full-length. It is apparent that hybridization also comprises hybridization with a polynucleotide comprising a general codon in consideration of codon degeneracy.

Whether or not any two polynucleotide or polypeptide sequences have homology, similarity, or identity may be determined using known computer algorithms such as the "FASTA" program, for example, using default parameters as in Pearson et al (1988) [Proc. Natl. Acad. Sci. USA 85]: 2444. Alternatively, it may be determined using Needleman-Wunsch algorithm (Needleman and Wunsch, 1970, J. Mol. Biol. 48: 443-453) as performed in the Needleman program of the EMBOSS package (EMBOSS: The European Molecular Biology Open Software Suite, Rice et al., 2000, Trends Genet. 16: 276-277) (version 5.0.0 or later) (comprising GCG program package ((Devereux, J., et al, Nucleic Acids Research 12: 387 (1984)), BLASTP, BLASTN, FASTA (Atschul, [S.] [F.,] [ET AL, J MOLEC BIOL 215]: 403 (1990); Guide to Huge Computers, Martin J. Bishop, [ED.,] Academic Press, San Diego,1994, and [CARILLO et al.](1988) SIAM J Applied Math 48: 1073). For example, BLAST of the National Center for Biotechnology Information or ClustalW may be used to determine the homology, similarity, or identity.

The homology, similarity, or identity between polynucleotides or polypeptides may be determined by comparing sequence information using the GAP computer program as introduced by, for example, Needleman et al. (1970), J Mol Biol. 48:443, as disclosed by Smith and Waterman, Adv. Appl. Math (1981) 2:482. Briefly, the GAP program defines the homology, similarity, or identity as the value obtained by dividing the number of similarly aligned symbols (i.e., nucleotides or amino acids) by the total number of the symbols in the shorter of the two sequences. The default parameters for the GAP program may comprise: (1) unitary matrices (comprising a value 1 for identity and a value 0 for non-identity), PAM Matrix (see those described by Schwartz and Dayhoff, eds., Atlas Of Protein Sequence And Structure, National Biomedical Research Foundation (1978)), and the weighted comparison matrix of Gribskov et al(1986) Nucl. Acids Res. 14: 6745 (or EDNAFULL (EMBOSS version of NCBI NUC4.4) substitution matrix); (2) a penalty of 3.0 for each gap and an additional 0.10 penalty for each symbol in each gap (or a gap open penalty of 10 and a gap extension penalty of 0.5); and (3) no penalty for end gaps.

Further, whether or not any two polynucleotide or polypeptide sequences have a homology, similarity or identity may be identified by comparing the sequences in a Southern hybridization experiment under stringent conditions as defined, and appropriate hybridization conditions defined are within the skill of the art, and may be determined by a method well known to those skilled in the art (e.g., J. Sambrook et al., Molecular Cloning, A Laboratory Manual; F.M. Ausubel et al., Current Protocols in Molecular Biology, John Wiley & Sons, Inc., New York), but is not limited thereto.

As used herein, the term "stringent conditions" means conditions that enable specific hybridization between polynucleotides. These conditions are specifically described in documents (see Sambrook et al., supra, 9.50-9.51, 11.7-11.8). Examples thereof comprise conditions in which polynucleotides having higher homology or identity, i.e., polynucleotides having 60% or more, 70% or more, 75% or more, 80% or more, 85% or more, 90% or more, 95% or more, 96% or more, 97% or more, 98% or more, or 99% or more homology or identity are hybridized with each other while polynucleotides having lower homology or identity are not hybridized with each other, or conditions in which washing is performed once, specifically, twice to three times at a salt concentration and temperature equivalent to 60°C, 1XSSC, 0.1% SDS, specifically 60°C, 0.1XSSC, 0.1% SDS, more specifically, 68°C, 0.1XSSC, 0.1% SDS, which are washing conditions for common Southern hybridization.

The hybridization requires that two nucleotides have complementary sequences, although mismatches between bases are allowed depending on the stringency of hybridization. The term "complementary" is used to describe the relation between nucleotide bases capable of being hybridized with each other. For example, with regard to DNA, adenine is complementary to thymine and cytosine is complementary to guanine. Hence, the polynucleotide of the present disclosure may also comprise substantially similar nucleotide sequences as well as isolated nucleic acid fragments that are complementary to the entire sequence.

For example, polynucleotides having a homology or identity to the polynucleotide of the present disclosure may be detected using the hybridization conditions comprising a hybridization step at a Tm value of 55°C under the above-described conditions. Further, the Tm value may be 60°C, 63°C, or 65°C, but is not limited thereto, and may be appropriately adjusted by those skilled in the art depending on the purpose thereof.

The appropriate stringency to hybridize the polynucleotide depends on the length and degree of complementarity of the polynucleotide, and the variables are well known in the art(e.g., J. Sambrook et al., supra).

As used herein, the term "L-citrulline" refers to an L-amino acid having a chemical formula of H₂NC(O)NH(CH₂)₃CH(NH₂)CO₂H.

As used herein, the term "microorganism (or strain)" comprises all of wild-type microorganisms, or microorganisms in which genetic modification naturally or artificially occurs, and it may be a microorganism in which a specific mechanism is weakened or enhanced due to insertion of a foreign gene or an activity enhancement or inactivation of an endogenous gene, and it may be a microorganism comprising genetic modification for the production of a desired polypeptide, protein, or product. As used herein, the "microorganism" and "strain" have the same meaning and may be used interchangeably without limitation.

As used herein, the term "microorganism having the L-citrulline-producing ability" refers to a prokaryotic or eukaryotic microbial strain capable of producing an L-citrulline within a living organism, and may comprise both a microorganism prepared by providing the L-citrulline-producing ability for a parent strain without the L-citrulline-producing ability, or a microorganism inherently having the L-citrulline-producing ability. The L-citrulline-producing ability may be conferred or enhanced by species improvement.

For example, the microorganism having the L-citrulline-producing ability of the present disclosure may be a microorganism (e.g., recombinant strain), in which the activity of the protein comprising the amino acid sequence of SEQ ID NO: 1 is weakened, as compared to its endogenous activity, but is not limited thereto.

As used herein, the term "unmodified microorganism" does not exclude strains comprising a mutation that may occur naturally in microorganisms, and may refer to a wild-type strain or a natural strain itself or may refer to a strain before the trait is changed by genetic variation due to natural or artificial factors. The "unmodified microorganism" may be used interchangeably with a "strain before being modified", "microorganism before being modified", "unvaried strain", "unmodified strain", "unvaried microorganism", "parent strain before being modified", "wild-type microorganism", "reference microorganism", or "standard microorganism". In the present disclosure, the unmodified microorganism may refer to a strain, in which the activity of the protein comprising the amino acid sequence of SEQ ID NO: 1 of the present disclosure is not weakened, as compared to its endogenous activity, or the activity of the protein comprising the amino acid sequence of SEQ ID NO: 1 has not yet been weakened, as compared to its endogenous activity, but is not limited thereto. In the present disclosure, the unmodified microorganism may be a microorganism comprising the amino acid sequence consisting of SEQ ID NO: 1 or the polynucleotide consisting of SEQ ID NO: 2, but is not limited thereto.

With respect to the objects of the present disclosure, the unmodified microorganism of the present disclosure may be a microorganism endogenously comprising
i) the protein consisting of the amino acid sequence of SEQ ID NO: 1, or a protein consisting of an amino acid sequence having at least 80%, 85%, 90%, 95%, 96%, 97%, 98%, 99%, 99.5%, 99.7%, or 99.9% or more homology or identity to the amino acid sequence of SEQ ID NO: 1; and/or
ii) a polynucleotide sequence capable of encoding a protein comprising an amino acid sequence having at least 80% homology to SEQ ID NO: 1, the nucleotide sequence of SEQ ID NO: 2, or a nucleotide sequence having 80% or more, 85% or more, 90% or more, 95% or more, 96% or more, 97% or more, 98% or more, and less than 100% homology or identity to the nucleotide sequence of SEQ ID NO: 2.

With respect to the objects of the present disclosure, the microorganism of the present disclosure may comprise any microorganism capable of producing a desired L-citrulline, in which the activity of the protein comprising the amino acid sequence of SEQ ID NO: 1 is weakened, as compared to its endogenous activity. For example, the microorganism of the present disclosure may be a genetically modified microorganism or a recombinant microorganism, characterized in that the L-citrulline-producing ability is increased by weakening the activity of the protein comprising the amino acid sequence of SEQ ID NO: 1, as compared to its endogenous activity, but is not limited thereto. Specifically, the recombinant strain in which the L-citrulline-producing ability is increased may be a microorganism in which the L-citrulline-producing ability is increased, as compared to that of the natural wild-type microorganism or the unmodified microorganism having the endogenous activity of the protein comprising the amino acid sequence of SEQ ID NO: 1, but is not limited thereto.

For example, the microorganism having the L-citrulline-producing ability may be a prokaryotic or eukaryotic microbial strain capable of producing L-citrulline within a living organism, and may comprise both a microorganism inherently having the L-citrulline-producing ability or a microorganism prepared by providing the L-citrulline-producing ability for a parent strain without the L-citrulline-producing ability due to the activity of the protein comprising the amino acid sequence of SEQ ID NO: 1 of the present disclosure which is weakened, as compared to its endogenous activity. The L-citrulline-producing ability may be conferred or enhanced by species improvement.

The microorganism of the present disclosure may comprise any microorganism in which the activity of the protein comprising the amino acid sequence of SEQ ID NO: 1 is weakened, as compared to its endogenous activity, by various known methods.

The "endogenous activity" means the activity of a specific polypeptide originally possessed by a parent strain before the trait is changed or an unmodified microorganism, when the trait is changed by genetic variation due to natural or artificial factors. This may be used interchangeably with "activity before modification".

As used herein, the term "weakening" of the polypeptide activity has a concept encompassing all of the weakening of activity or the absence of activity, as compared to the endogenous activity. The weakening may be used interchangeably with terms such as deficiency (inactivation), deletion, disruption, down-regulation, decrease, attenuation, repression, reduction, etc.

For example, the weakening of the polypeptide activity, which is a state of being not completely inactivated by deletion even though the polypeptide activity is exhibited in a host cell (microorganism), may comprise a case where the polypeptide activity is reduced, as compared to the endogenous activity or the activity before modification; or a case where the polypeptide is not expressed at all, as compared to a host cell (microorganism) before the trait is changed, or an unmodified microorganism, or the activity is absent or reduced (inactivated) even though expressed. The weakening may also comprise a case where the activity of the polypeptide itself is weakened or eliminated due to variation of the polynucleotide encoding the polypeptide, etc., as compared to the activity of the polypeptide originally possessed by the microorganism, a case where the overall polypeptide activity level in the cell is low due to inhibition of the expression of the gene encoding the polypeptide or due to inhibition of translation into the polypeptide, as compared to that of the native strain, a case where the gene is not expressed at all, and a case where the polypeptide activity is absent even when the gene is expressed.

The fact that the activity of the polypeptide is weakened as compared to the endogenous activity means that the activity of the polypeptide is lowered as compared to the activity of the specific polypeptide originally possessed by a parent strain before the trait is changed or an unmodified microorganism. The weakening of the activity of the polypeptide may be confirmed by weakening in the degree of activity and the expression level of the corresponding polypeptide or weakening in the amount of the product released from the corresponding polypeptide.

For example, the weakening indicates that the activity of the protein comprising the amino acid sequence of SEQ ID NO: 1 may be less than about 100%, about 90% or less, about 80% or less, about 70% or less, about 60% or less, about 50% or less, about 40% or less, about 30% or less, about 20% or less, about 10% or less, about 5% or less, or 0% of that of the protein comprising the amino acid sequence of SEQ ID NO: 1 of a parent strain before the trait is changed or an unmodified microorganism, but is not limited thereto.

For example, the inactivation may mean that the protein comprising the amino acid sequence of SEQ ID NO: 1 is not expressed at all, as compared to an unmodified microorganism, or even though expressed, its activity is absent or weakened.

Such weakening of the activity of the polypeptide may be performed by any method known in the art, but the method is not limited thereto, and the weakening may be achieved by applying various methods well known in the art (e.g., Nakashima N et al., Bacterial cellular engineering by genome editing and gene silencing. Int J Mol Sci. 2014;15(2):2773-2793, Sambrook et al. Molecular Cloning 2012, etc.).

Specifically, the weakening of the activity of the polypeptide of the present disclosure may be:
1) deletion of the entirety or a part of the gene encoding the polypeptide;
2) modification of an expression regulatory region (or expression regulatory sequence) to weaken expression of the gene encoding the polypeptide;
3) modification of an amino acid sequence constituting the polypeptide to eliminate or weaken the activity of the polypeptide (e.g., deletion/substitution/addition of one or more amino acids in the amino acid sequence);
4) modification of a polynucleotide sequence encoding the polypeptide to eliminate or weaken the activity of the polypeptide (e.g., deletion/substitution/addition of one or more nucleic acid bases in a nucleotide sequence of the polypeptide gene to encode the polypeptide that has been modified to eliminate or weaken the activity of the polypeptide):
5) modification of a nucleotide sequence of a start codon or a 5'-UTR region of the gene encoding the polypeptide;
6) introduction of an antisense oligonucleotide (e.g., antisense RNA) that complementarily binds to the transcript of the gene encoding the polypeptide;
7) addition of a sequence complementary to the Shine-Dalgarno sequence of the gene encoding the polypeptide to the upstream of the Shine-Dalgarno sequence in order to form a secondary structure that makes the attachment of ribosomes impossible;
8) addition of a promoter to be transcribed in the opposite direction to the 3' end of the open reading frame (ORF) of the polynucleotide sequence encoding the polypeptide (reverse transcription engineering, RTE);
9) controlling of intracellular localization of the protein (polypeptide); or
10) a combination of two or more selected from 1) to 9), but is not particularly limited thereto.

For example,
1) the deletion of a part or the entirety of the gene encoding the polypeptide may be removal of the entire polynucleotide encoding the endogenous target polypeptide in the chromosome, replacement with a polynucleotide in which some nucleotide sequences are deleted, or replacement with a marker gene.

Such deletion of a part or the entirety of the polynucleotide may be performed by a method of deleting the polynucleotide by homologous recombination using a vector for chromosome insertion in the microorganism, or a method of inducing mutations with light, such as ultraviolet light, or chemicals, and then selecting, from the obtained mutants, a strain in which the target gene is deleted, but is not limited thereto. The method of deleting a part or the entirety of the gene may comprise a method of using a DNA recombinant technology. For example, deletion of a part or the entirety of the gene may be achieved by injecting a nucleotide sequence or vector comprising a nucleotide sequence with homology to the target gene into the microorganism to cause homologous recombination. The nucleotide sequence or vector to be injected may comprise a dominant selection marker, but is not limited thereto.

Further, 2) the modification of the expression regulatory sequence may be occurrence of variation on the expression regulatory region (or expression regulatory sequence) due to deletion, insertion, non-conservative or conservative substitution, or a combination thereof, or replacement with a sequence having weaker activity. The expression regulatory region comprises a promoter, an operator sequence, a sequence encoding a ribosome binding site, and a sequence regulating the termination of transcription and translation, but is not limited thereto.

Further, 3) and 4) the modification of the amino acid sequence or polynucleotide sequence may be occurrence of variation on the sequence due to deletion, insertion, or non-conservative or conservative substitution of the amino acid sequence of the polypeptide or the polynucleotide sequence encoding the polypeptide, or a combination thereof, or replacement with an amino acid sequence or a polynucleotide sequence improved to have weaker activity or an amino acid sequence or a polynucleotide sequence improved to be inactive so that the activity of the polypeptide is weakened, but is not limited thereto. For example, expression of the gene may be inhibited or weakened by introducing variation into the polynucleotide sequence and forming a stop codon, but is not limited thereto.

Further, 5) the modification of a nucleotide sequence of a start codon or a 5'-UTR region of the gene encoding the polypeptide may be, for example, substitution with another start codon having a lower polypeptide expression rate, as compared to an endogenous start codon, but is not limited thereto.

6) The introduction of an antisense oligonucleotide (e.g., antisense RNA) that complementarily binds to the transcript of the gene encoding the polypeptide may refer to documents, for example, [Weintraub, H. et al., Antisense-RNA as a molecular tool for genetic analysis, Reviews - Trends in Genetics, Vol. 1(1) 1986].

7) The addition of a sequence complementary to the Shine-Dalgarno sequence of the gene encoding the polypeptide to the upstream of the Shine-Dalgarno sequence in order to form a secondary structure that makes the attachment of ribosomes impossible may be to make mRNA translation impossible or to slow down the mRNA translation rate.

8) The addition of a promoter to be transcribed in the opposite direction to the 3' end of the open reading frame (ORF) of the polynucleotide sequence encoding the polypeptide (reverse transcription engineering, RTE) may be to weaken the activity by making an antisense nucleotide complementary to the transcript of the gene encoding the polypeptide.

9) The controlling of intracellular localization of the protein (polypeptide) may mean targeting the protein (polypeptide) to a specific organelle within cells or to a specific intracellular space, for example, targeting to the periplasm or cytoplasm through the addition or removal of a leader sequence that functions in targeting the protein (polypeptide), but is not limited thereto.

Such weakening of the activity of the polypeptide may mean that the activity or concentration (expression level) of the corresponding polypeptide is weakened, based on the activity or concentration of the polypeptide expressed in a wild-type or a microbial strain before modification, or that the amount of a product produced from the corresponding polypeptide is decreased, but is not limited thereto.

The modification of a part or the entirety of the polynucleotide in the microorganism of the present disclosure may be induced by (a) homologous recombination using a vector for chromosomal insertion in a microorganism or genome editing using engineered nuclease (e.g., CRISPR-Cas9), and/or (b) the treatment with light, such as ultraviolet light and radiation, and/or chemicals, but is not limited thereto. The method of modifying a part or the entirety of the gene may comprise a method by DNA recombinant technology. For example, a nucleotide sequence or vector comprising a nucleotide sequence homologous to a target gene may be introduced into the microorganism to bring about homologous recombination, resulting in deletion in a part or the entirety of the gene. The nucleotide sequence or vector to be introduced may comprise a dominant selection marker, but is not limited thereto.

For example, the recombinant microorganism having the L-citrulline-producing ability of the present disclosure may comprise any microorganism that is transformed with a vector to have the weakened activity of the protein comprising the amino acid sequence of SEQ ID NO: 1 of the present disclosure, thereby capable of producing L-citrulline.

The vector of the present disclosure may comprise a DNA construct comprising a nucleotide sequence of a polynucleotide encoding a target polypeptide which is operably linked to a suitable expression regulatory region (or expression regulatory sequence) so that the target polypeptide may be expressed in a suitable host. The expression regulatory region may comprise a promoter capable of initiating transcription, any operator sequence for controlling the transcription, a sequence encoding a suitable mRNA ribosome binding site, and a sequence controlling termination of transcription and translation. The vector may be transformed into a suitable host cell, and then replicated or function independently of the host genome, or may be integrated into the genome itself.

The vector used in the present disclosure is not particularly limited, but any vector known in the art may be used. Examples of commonly used vectors comprise natural or recombinant plasmids, cosmids, viruses, and bacteriophages. For example, pWE15, M13, MBL3, MBL4, IXII,ASHII,APII, t10, t11, Charon4A, and Charon21A, etc. may be used as a phage vector or a cosmid vector, and pDZ system, pBR system, pUC system, pBluescript II system, pGEM system, pTZ system, pCL system, pSK system, pSKH system, and pET system, etc. may be used as a plasmid vector. Specifically, pDZ, pDC, pDCM2, pACYC177, pACYC184, pCL, pSK, pSKH130, pECCG117, pUC19, pBR322, pMW118, pCC1BAC vector, etc. may be used.

For example, a polynucleotide encoding a target polypeptide may be inserted into a chromosome through a vector for intracellular chromosome insertion. Insertion of the polynucleotide into the chromosome may be performed by any method known in the art, for example, homologous recombination, but is not limited thereto. The vector may further comprise a selection marker for checking the chromosome insertion. The selection marker is for selecting the cells transformed with vectors, i.e., for checking the insertion of a target nucleic acid molecule, and markers that confer selectable phenotypes such as drug resistance, auxotrophy, resistance to cytotoxic agents, or expression of surface polypeptides may be used. In an environment treated with a selective agent, only cells expressing the selection marker may survive or exhibit other phenotypic traits, and thus transformed cells may be selected.

As used herein, the term "transformation" means that a vector comprising a polynucleotide encoding a target polypeptide is introduced into a host cell or a microorganism so that the polypeptide encoded by the polynucleotide may be expressed in the host cell. The transformed polynucleotide may be located regardless of the position, either by being inserted into the chromosome of the host cell or located outside the chromosome as long as it may be expressed in the host cell. Further, the polynucleotide comprises DNA and/or RNA encoding a target polypeptide. The polynucleotide may be introduced in any form as long as it may be introduced into a host cell and expressed. For example, the polynucleotide may be introduced into a host cell in the form of an expression cassette, which is a gene construct comprising all elements required for self-expression. The expression cassette may usually comprise a promoter operably linked to the polynucleotide, a transcription termination signal, a ribosome binding site, and a translation termination signal. The expression cassette may be in the form of an expression vector capable of self-replicating. Further, the polynucleotide may be introduced into a host cell in its own form and operably linked to a sequence required for expression in the host cell, but is not limited thereto.

As used herein, the term "operably linked" refers to a configuration of placing a regulatory sequence to an appropriate position to direct expression of a coding sequence. Thus, "operably linked" comprises an attachment or linking between a regulatory region of a functional domain having a known or desired activity such as a promoter, a terminator, a signal sequence, or an enhancer, and a target (gene or polypeptide) such that the expression, secretion, or function of the target (gene or polypeptide) may be regulated in accordance with the known or desired activity. For example, the term means that the polynucleotide sequence is functionally linked to a promoter sequence that initiates and mediates transcription of the polynucleotide encoding the target variant polypeptide of the present disclosure.

As used herein, the term "expression" comprises any step involved in production of a polypeptide, e.g., transcription, post-transcriptional modification, translation, post-translational modification, and secretion, but is not limited thereto.

As used herein, the term "expression vector" refers to a linear or circular nucleic acid molecule comprising a coding sequence and an operably linked regulatory sequence for expression thereof.

As used herein, the term "regulatory sequence" refers to a polynucleotide sequence required for the expression of a coding sequence. Each regulatory sequence may be native (derived from the same origin) or foreign (derived from different genes) to the coding sequence. Examples of the regulatory sequence may comprise a leader sequence, a polyadenylation sequence, a propeptide sequence, a promoter, a signal peptide sequence, an operator sequence, a ribosome binding siteencoding sequence, and a sequence terminating transcription and translation. A minimum unit of the regulatory sequence may comprise a promoter and a sequence terminating transcription and translation.

With regard to a cell, polynucleotide, polypeptide, or vector, the term "recombinant" used herein refers to the cell, polynucleotide, polypeptide, or vector modified by introduction of a heterologous nucleic acid or polypeptide or alteration of a native polynucleotide or polypeptide, or a cell derived from the modified cell. Thus, for example, recombinant cells may express genes that are not found within the native (non-recombinant) form of the cells, or may express native genes that are otherwise abnormally expressed, under expressed or not expressed at all.

The microorganism of the present disclosure may comprise any microorganism with the weakened activity of the protein comprising the amino acid sequence of SEQ ID NO: 1, as compared to its endogenous activity, by various known methods.

In one specific embodiment, the microorganism with the weakened activity of the protein comprising the amino acid sequence of SEQ ID NO: 1 of the present disclosure, as compared to its endogenous activity, may be a microorganism in which the polynucleotide encoding the protein comprising the amino acid sequence of SEQ ID NO: 1 is deleted or modified, but is not limited thereto. Specifically, the microorganism may be a microorganism in which the activity of the protein comprising the amino acid sequence of SEQ ID NO: 1 is absent due to deletion of the nucleotide sequence of SEQ ID NO: 2, but is not limited thereto.

For example, the microorganism having the increased L-citrulline-producing ability of the present disclosure may be a microorganism in which the L-citrulline-producing ability is increased, as compared to that of an unmodified microorganism, but is not limited thereto. For example, the unmodified microorganism, which is a subject strain for comparing whether or not the L-citrulline-producing ability is increased, may be C. gl::argR*_argG* strain, but is not limited thereto.

For example, the microorganism having the increased L-citrulline-producing ability may have an increased L-citrulline-producing ability of about 1% or more, specifically, about 1% or more, about 2.5% or more, about 5% or more, about 6% or more, about 7% or more, about 8% or more, about 9% or more, about 10% or more, about 11% or more, about 12% or more, about 13%or more, about 14% or more, about 15% or more, about 16% or more, about 17% or more, about 18% or more, about 19% or more, about 20% or more, or about 21% or more (the upper limit is not particularly limited, but may be, for example, about 200% or less, about 150% or less, about 100% or less, about 50% or less, about 45% or less, about 40% or less, about 35% or less, about 30% or less, about 25% or less, or about 22% or less), as compared to that of the parent strain before modification or unmodified microorganism, but the increased amount is not limited thereto as long as the production ability has an increased amount of a + value, as compared to the production ability of the parent strain before modification or the unmodified microorganism. In another example, the recombinant strain having the increased L-citrulline-producing ability may have an increased L-citrulline-producing ability of about 1.1 times or more, about 1.11 times or more, about 1.12 times or more, about 1.13 times or more, about 1.14 times or more, about 1.15 times or more, about 1.16 times or more, about 1.17 times or more, about 1.18 times or more, about 1.19 times or more, about 1.2 times or more, or about 1.21 times or more (the upper limit is not particularly limited, but may be, for example, about 10 times or less, about 5 times or less, about 3 times or less, about 2 times or less, about 1.5 times or less, about 1.4 times or less, about 1.3 times or less, or about 1.25 times or less), as compared to that of the parent strain before modification or the unmodified microorganism, but is not limited thereto.

For example, the microorganism having the L-citrulline-producing ability may be either a prokaryotic cell or a eukaryotic cell, specifically, a prokaryotic cell. The prokaryotic cell may comprise, for example, microbial strains of the genus *Escherichia* (*Escherichia* sp.), the genus *Erwinia* (*Erwinia* sp.), the genus *Serratia* (*Serratia* sp.), the genus *Providencia* (*Providencia* sp.), the genus *Corynebacterium* (*Corynebacterium* sp.), the genus *Pseudomonas* (*Pseudomonas* sp.), the genus *Leptospira* (*Leptospira* sp.), the genus *Salmonella* (*Salmonella* sp.), the genus *Brevibacteria* (*Brevibacteria* sp.), the genus *Hypomononas* (*Hypomononas* sp.), the genus *Chromobacterium* (*Chromobacterium* sp.), and the genus *Norcardia* (*Norcardia* sp.), or microbial strains of fungi or yeasts. Specifically, the microorganism may be a microbial strain of the genus *Escherichia,* the genus *Corynebacterium,* the genus *Leptospira,* or a yeast. More specifically, the microorganism may be a microbial strain of the genus *Corynebacterium.*

With regard to the microorganism according to any one of the aforementioned specific embodiments, the microorganism of the present disclosure may be a microorganism of the genus *Corynebacterium.*

For example of the present disclosure, the microorganism of the present disclosure may be *Corynebacterium glutamicum, Corynebacterium crudilactis, Corynebacterium deserti, Corynebacterium efficiens, Corynebacterium callunae, Corynebacterium stationis, Corynebacterium singulare, Corynebacterium halotolerans, Corynebacterium striatum, Corynebacterium ammoniagenes, Corynebacterium pollutisoli, Corynebacterium imitans, Corynebacterium testudinoris,* or *Corynebacterium flavescens.* Specifically, the microorganism of the present disclosure may be a microorganism of the genus *Corynebacterium,* more specifically, *Corynebacterium glutamicum* or *Corynebacterium stationis,* but is not limited thereto.

Meanwhile, the microorganism having the L-citrulline-producing ability of the present disclosure may comprise all of a natural wild-type microorganism itself, a microorganism that has the improved L-citrulline-producing ability by enhancing or weakening the activities of genes related to the L-citrulline production mechanism, or a microorganism that has the improved L-citrulline-producing ability by introducing or enhancing the activity of a foreign gene.

As used herein, the term "culturing" refers to growing the strain of the present disclosure in appropriately adjusted environment conditions. The culturing procedure of the present disclosure may be performed according to appropriate media or culture conditions known in the art. Such culturing procedure may be easily adjusted according to the selected strain by a person skilled in the art. Specifically, the culturing may be in a batch type, a continuous type, and/or a fed-batch type, but is not limited thereto.

As used herein, the "medium" refers to a mixture comprising, as main ingredients, nutrient materials required for culturing the microorganism of the present disclosure, wherein the medium supplies nutrient materials comprising water essential for survival and growth, growth factors, etc. Specifically, as for the media and other culture conditions used for culturing the strain of the present disclosure, any medium that is used for usual culture of microorganisms may be used without particular limitation. However, the microorganism of the present disclosure may be cultured under aerobic conditions in a common medium comprising appropriate carbon sources, nitrogen sources, phosphorus sources, inorganic compounds, amino acids, and/or vitamins, etc., while controlling the temperature, pH, etc. For example, a culture medium for strains of the genus *Corynebacterium* may be found in a literature ["Manual of Methods for General Bacteriology" by the American Society for Bacteriology (Washington D.C., USA, 1981)].

In the present disclosure, the carbon source may comprise carbohydrates, such as glucose, saccharose, lactose, fructose, sucrose, maltose, etc.; sugar alcohols, such as mannitol, sorbitol, etc.; organic acids, such as pyruvic acid, lactic acid, citric acid, etc.; and amino acids, such as glutamic acid, methionine, lysine, etc. In addition, natural organic nutrient sources may be used, such as starch hydrolysates, molasses, blackstrap molasses, rice bran, cassava, bagasse, and corn steep liquor, and specifically, carbohydrates, such as glucose and sterile pretreated molasses (i.e., molasses converted to reduced sugars) may be used, and appropriate amounts of other carbon sources may be variously used without limitation. These carbon sources may be used alone or in a combination of two or more thereof, but are not limited thereto.

As for the nitrogen sources, inorganic nitrogen sources, such as ammonia, ammonium sulfate, ammonium chloride, ammonium acetate, ammonium phosphate, ammonium carbonate, ammonium nitrate, etc.; amino acids, such as glutamic acid, methionine, glutamine, etc.; and organic nitrogen sources, such as peptone, NZ-amine, meat extracts, yeast extracts, malt extracts, corn steep liquor, casein hydrolysates, fishes or decomposition products thereof, defatted soybean cake or decomposition products thereof, etc. may be used. These nitrogen sources may be used alone or in a combination of two or more thereof, but are not limited thereto.

The phosphate sources may comprise potassium phosphate monobasic, potassium phosphate dibasic, and sodium-comprising salts corresponding thereto. As for inorganic compounds, sodium chloride, calcium chloride, iron chloride, magnesium sulfate, iron sulfate, manganese sulfate, calcium carbonate, etc. may be used, and in addition, amino acids, vitamins, and/or suitable precursors may be comprised. These components or precursors may be added to the medium in a batch or continuous manner. However, the present disclosure is not limited thereto.

Further, pH of the medium may be adjusted by adding compounds, such as ammonium hydroxide, potassium hydroxide, ammonia, phosphoric acid, sulfuric acid, etc., to the medium in an appropriate manner during the culture of the microorganism of the present disclosure. In addition, an anti-foaming agent, such as a fatty acid polyglycol ester, may be used to suppress foam formation during the culture. In addition, oxygen or oxygen-comprising gas may be injected into the medium to maintain the aerobic state of the medium, or no gas may be injected, or nitrogen, hydrogen, or carbon dioxide gas may be injected to maintain the anaerobic or nonaerobic state, but is not limited thereto.

In the culture of the present disclosure, the culture temperature may be maintained at 27°C to 37°C, specifically, at 30°C to 33°C, and the culture may be performed for about 20 hours to 120 hours, but is not limited thereto.

As used herein, the term "culture" refers to a culture liquid obtained by culturing a specific microorganism in a culture medium, a concentrated culture liquid, a dry product of the culture liquid, a culture filtrate, a concentrated culture filtrate, or a dry product of the culture filtrate, and the culture liquid refers to those comprising the specific microorganism, and the culture filtrate refers to those substantially not comprising the specific microorganism (which means that the specific microorganism to be separated by filtration, etc. are substantially excluded, which does not mean that the microorganism is completely excluded from the filtrate.). The formulation of the culture is not limited, but it may be, for example, a liquid, an emulsion, or a solid. Specifically, with respect to the objects of the present disclosure, the culture may comprise L-citrulline.

As used herein, the term "fermentation" refers to a process whereby microorganisms break down organic materials using their own enzymes, excluding putrefaction. Fermentation and putrefaction proceed by similar processes. However, when useful substances are produced as a result of decomposition, it is called fermentation, and when bad smells or harmful substances are produced, it is called putrefaction.

In the present disclosure, the method of obtaining the fermentation product from the strain is not particularly limited, and the fermentation product may be obtained according to a method commonly used in the art or similar art.

As used herein, the term "fermentation product" comprises not only a fermented material itself, but also all types of materials comprising the fermentation product generated from the strain, such as a culture medium of a strain in which the strain and the culture coexist, a fermentation product obtained by filtering the strain from the culture medium, a fermentation product obtained by sterilizing the strain from the culture medium and filtering the same, an extract obtained by extracting the fermentation product or the culture medium comprising the same, a dilution obtained by diluting the fermentation product or the extract thereof, a concentrate, a dry product obtained by drying the fermentation product or the extract thereof, a lysate obtained by collecting and disrupting cells of the strain, etc.

With regard to the method of the present disclosure, the culturing of the microorganism may be performed using any culturing conditions and culturing method known in the art. Such culturing procedure may be easily adjusted for use by a person skilled in the art according to the selected strain.

The L-citrulline produced by culturing of the present disclosure may be released into the medium or may remain in cells.

In one specific embodiment, the method of producing L-citrulline of the present disclosure may further comprise the steps of preparing the microorganism of the present disclosure, preparing a medium for culturing the strain, or a combination of these steps (regardless of the order, in any order), for example, before the culturing step.

The method of producing L-citrulline of the present disclosure may further comprise the step of recovering a desired substance, specifically, L-citrulline from the cultured microorganism, a culture of the microorganism, a fermentation product of the microorganism, or the culture medium. The recovering step may be further comprised after the culturing step.

The recovering may be collecting the desired L-citrulline by using an appropriate method known in the art according to the method of culturing the microorganism of the present disclosure, for example, a batch, continuous, or fed-batch type culture. For example, centrifugation, filtration, treatment with a crystallized protein precipitating agent (salting-out), extraction, sonication, ultrafiltration, dialysis, various types of chromatography, such as molecular sieve chromatography (gel filtration), adsorption chromatography, ion exchange chromatography, affinity chromatography, HPLC, and a combination of these methods may be used, and a desired substance, specifically, L-citrulline may be recovered from the medium or microorganism by using an appropriate method known in the art.

In addition, the method of producing L-citrulline of the present disclosure may further comprise a purification step. The purification may be performed by using an appropriate method known in the art. In an exemplary embodiment, when the method of producing L-citrulline of the present disclosure comprises both the recovering step and the purification step, the recovering step and the purification step may be performed continuously or discontinuously regardless of the order, or may be performed simultaneously or integrated into one step, but is not limited thereto.

With regard to the method of the present disclosure, the weakening of the activity of the protein comprising the amino acid sequence of SEQ ID NO: 1 and L-citrulline, etc. are as described in other aspects.

Another aspect of the present disclosure provides a composition for producing L-citrulline, the composition comprising the microorganism of the genus *Corynebacterium* having the L-citrulline-producing ability, in which the activity of the protein comprising the amino acid sequence of SEQ ID NO: 1 of the present disclosure is weakened, as compared to its endogenous activity, a culture of the microorganism, a fermentation product of the microorganism, or a combination of two or more thereof.

The composition of the present disclosure may further comprise any appropriate excipient that is usually used in the composition for producing L-citrulline, and examples of the excipient may comprise a preserving agent, a wetting agent, a dispersing agent, a suspending agent, a buffer, a stabilizing agent, an isotonic agent, etc., but are not limited thereto.

In a specific embodiment, each component present in the composition of the present disclosure may be comprised in a microbially effective amount, or in an amount that may be appropriately present in the composition for production.

With regard to the composition of the present disclosure, the weakening of the activity of the protein comprising the amino acid sequence of SEQ ID NO: 1 and L-citrulline, etc. are as described in other aspects.

Still another aspect of the present disclosure provides use of the microorganism of the genus *Corynebacterium* having the L-citrulline-producing ability, in which the activity of the protein comprising the amino acid sequence of SEQ ID NO: 1 of the present disclosure is weakened, as compared to its endogenous activity, in producing L-citrulline.

With regard to the use of the present disclosure, the weakening of the activity of the protein comprising the amino acid sequence of SEQ ID NO: 1 and L-citrulline, etc. are as described in other aspects.

### [Mode for Carrying Out the Invention]

Hereinafter, the present disclosure will be described in more detail by way of exemplary embodiments. However, the following exemplary embodiments are only preferred embodiments for illustrating the present disclosure, and thus are not intended to limit the scope of the present disclosure thereto. Meanwhile, technical matters not described in the present specification may be sufficiently understood and easily implemented by those skilled in the technical field of the present disclosure or similar technical fields.

### Example 1: Construction of recombinant vector for deletion of NCgl2383 gene

To examine the relationship between the inactivation of a protein comprising an amino acid sequence of SEQ ID NO: 1 and the L-citrulline-producing ability, a recombinant plasmid was constructed to delete a gene comprising a nucleotide sequence of NCgl2383 on the chromosome of a *Corynebacterium* strain producing L-citrulline.

First, in order to construct the recombinant vector capable of deleting the gene on the chromosome of a microorganism of the genus *Corynebacterium,* four types of primers corresponding to SEQ ID NOS: 3, 4, 5, and 6 were synthesized.

In detail, in order to delete the ORF region (SEQ ID NO: 2) of the NCgl2383 gene, primer 3 (SEQ ID NO: 3), primer 4 (SEQ ID NO: 4), primer 5 (SEQ ID NO: 5), and primer 6 (SEQ ID NO: 6) were synthesized to have BamHI and Xbal restriction enzyme sites at the 5'- and 3'-ends. PCR was performed using the primers of SEQ ID NOS: 3, 4, 5, and 6 and chromosomal DNA of *Corynebacterium glutamicum* ATCC13869 as a template. As a result, it was confirmed that each 1000 bp of DNA fragments were amplified, which correspond to the upstream and downstream parts of the gene encoding the protein. PCR conditions were denaturation at 95°C for 10 minutes, followed by 30 cycles of denaturation at 95°C for 30 seconds, annealing at 55°C for 30 seconds, and polymerization at 72°C for 1 minute, and then polymerization at 72°C for 10 minutes. Thereafter, the PCR fragment was extracted using a gel purification kit (QIAGEN). The obtained recombinant arm gene fragment and a vector pDCM2 (WO WO2021-187781 A1) digested with BamHI and Xbal restriction enzymes were linked using the Gibson assembly method (DG Gibson et al., NATURE METHODS, VOL.6 NO.5, MAY 2009, NEBuilder HiFi DNA Assembly Master Mix), and then transformed into *E. coli* DH5α and plated on an LB solid medium comprising kanamycin (25 mg/l).

In order to select colonies transformed with the vector in which the target gene and pDCM2 were linked, PCR was performed using primers of SEQ ID NOS: 7 and 8. A plasmid was obtained from the selected colonies using a plasmid extraction method commonly known, and named pDCM2-ΔNCgl2383.

### Example 2: Preparation of L-citrulline-producing microorganism

To prepare an L-citrulline-producing microorganism, a vector for replacing glutamic acid at position 47 of a protein sequence of argR (ANU33619.1) with a stop codon was constructed. The genome of the wild-type *C*. *glutamicum* ATCC 13869 was used as a template to amplify a homologous recombinant A arm using a pair of primers of SEQ ID NOS: 9 and 10, and a homologous recombinant B arm using a pair of primers of SEQ ID NOS: 11 and 12. Thereafter, a plasmid was obtained in the same manner as Example 1, and this plasmid was named pDCM2-argR(E47*).

In order to prepare a microorganism with the further enhanced L-citrulline-producing ability, a vector for replacing phenylalanine at position 68 of a protein sequence of argG (ANU33620.1) with a stop codon was constructed. The genome of *C. glutamicum* ATCC13869 was used as a template to amplify a homologous recombinant A arm using a pair of primers of SEQ ID NOS: 13 and 14, and a homologous recombinant B arm using a pair of primers of SEQ ID NOS: 15 and 16. Thereafter, a plasmid was obtained using the method described above, and this plasmid was named pDCM2- argG(F68*).

The wild-type *C*. *glutamicum* ATCC 13869 was transformed with the constructed pDCM2-argR(E47*) vector by electroporation (Appl. Microbiol.Biotechnol. (1999) 52:541-545), and then through a secondary crossover process, a strain was obtained, in which the base sequence at position 139 of argR was replaced from guanine (g) to thymine (t), and the base sequence at position 47 was replaced with a stop codon. PCR and base sequence analysis were performed using a primer pair of SEQ ID NOS: 9 and 12 capable of amplifying the adjacent region comprising the position where the corresponding gene was inserted, and the corresponding genetic manipulation was confirmed. The microorganism thus obtained was named C. gl::argR*.

To prepare a microorganism with the further enhanced citrulline-producing ability in C. gl::argR*, the microorganism was obtained using the pDCM2-argG(F68*) vector by the method as described above. PCR and base sequence analysis were performed using a primer pair of SEQ ID NOS: 13 and 16 capable of amplifying the adjacent region comprising the position where the corresponding gene was inserted, and the corresponding genetic manipulation was confirmed. The microorganism thus obtained was named C. gl::argR*_argG*.

The primer sequences used are as in Table 1 below.

**[Table 1]**

| SEQ ID NO. | Name | Sequence (5' -> 3') |
|---|---|---|
| 9 | Primer 9 | CGGTACCCGGGGATCCCTCGTGCGG AATTCGTGGAG |
| 10 | Primer 10 | ATCCAGCAGCAATTCAGACA |
| 11 | Primer 11 | CTGAATTGCTGCTGGATTAAGGCATCG ATATCACCCA |
| 12 | Primer 12 | ATGCCTGCAGGTCGACCCTTCATTTTA AGTTCCTTG |
| 13 | Primer 13 | CGGTACCCGGGGATCCTTCATCGATA GGGTGGG |
| 14 | Primer 14 | GTACTCCTCAGCTTACTCATCCTTTGC ATCAACA |
| 15 | Primer 15 | AGTAAGCTGAGGAGTACTGCCTGCCA ACCATCAA |
| 16 | Primer 16 | ATGCCTGCAGGTCGACCGACTGGCTT GCCACCCT |

### Example 3: Preparation of NCgl2383 gene-deficient strain

Based on the C. gl::argR*_argG* strain which is an L-citrulline-producing strain of the genus *Corynebacterium,* a strain was prepared, in which the gene comprising the nucleotide sequence of SEQ ID NO: 2 was deleted.

In detail, the recombinant plasmid pDCM2-ΔNCgl2383 constructed in Example 1 was transformed into the L-citrulline-producing strain *Corynebacterium glutamicum* C. gl::argR*_argG* prepared in Example 2 by homologous recombination on the chromosome (van der Rest et al., Appl Microbiol Biotechnol 52:541-545, 1999).

Thereafter, a secondary recombination was performed on a solid plate medium comprising sucrose. The strain in which the gene was deleted was identified by PCR using primer 3 and primer 6 for the *Corynebacterium glutamicum* transformant in which the secondary recombination had been completed. The recombinant strain was named *Corynebacterium glutamicum* C. gl::argR*_argG*_ΔNCgl2383.

### Example 4: Evaluation of L-citrulline-producing ability of NCgl2383 gene-deficient strain

To analyze the L-citrulline-producing ability, the parent strain, *Corynebacterium glutamicum* C. gl::argR*_argG* strain, and the prepared *Corynebacterium glutamicum* C. gl::argR*_argG*_ΔNCgl2383 strain were cultured using the following method.

Each strain was inoculated into a 250 ml corner-baffled flask comprising 25 ml of a production medium below, and cultured at 33°C for 48 hours with shaking at 200 rpm. After the culture was completed, the cell mass and L-citrulline production amounts were measured using HPLC.

### <Production medium (pH 7.2)>

Raw sugar 50 g, (NH₄)₂SO₄ 30 g, Yeast extract 1 g, KH₂PO₄ 1.1 g, MgSO₄·7H₂O 1.2 g, L-arginine 0.2 g, Biotin 1 mg, Thiamine hydrochloride 5 mg, Calcium pantothenic acid 5 mg, Nicotinamide 15 mg, MnSO₄ 10 mg, FeSO₄ 10 mg, ZnSO₄ 0.5 mg, CuSO₄ 0.5 mg, CaCO₃ 30 g (based on 1 liter of distilled water)

The above experiment was repeated twice, and the culture results (mean value) are shown in Table 2 below.

**[Table 2]**

| | Strain | OD562 | Sugar consumption (g/L) | L-citrulline production amount (g/L) | Increase rate (%) of L-citrulline production | L-citrulline yield (%) |
|---|---|---|---|---|---|---|
| Control strain | C. gl::argR*_argG* | 60.9 | 50.0 | 2.8 | - | 5.5 |
| NCgl2383 | C. gl::argR*_argG*_ΔNCgl2383 gene-deficient strain | 58.6 | 50.0 | 3.4 | 21 | 6.9 |

As shown in Table 2, it was confirmed that the L-citrulline productivity of C. gl::argR*_argG*_ΔNCgl2383, which was obtained by deleting the NCgl2383 gene from the L-citrulline-producing strain *Corynebacterium glutamicum* C. gl::argR*_argG*, increased by 21% on average without affecting the cell body, as compared to the parent strain.

Accordingly, it was confirmed that the L-citrulline productivity may be improved by deleting the gene comprising the nucleotide sequence of SEQ ID NO: 2 in the microorganisms of the genus *Corynebacterium.*

Based on the above description, it will be understood by those skilled in the art that the present disclosure may be implemented in a different specific form without changing the technical spirit or essential characteristics thereof. In this regard, it should be understood that the above embodiment is not limitative, but illustrative in all aspects. The scope of the disclosure is defined by the appended claims rather than by the description preceding them, and therefore all changes and modifications that fall within metes and bounds of the claims, or equivalents of such metes and bounds are therefore intended to be embraced by the claims.

## Claims

1. A method of producing L-citrulline, the method comprising the step of culturing, in a medium, a microorganism of the genus *Corynebacterium* having an L-citrulline-producing ability, wherein the activity of a protein comprising an amino acid sequence of SEQ ID NO: 1 is weakened, as compared to its endogenous activity.

2. The method of claim 1, wherein the protein comprising the amino acid sequence of SEQ ID NO: 1 is encoded by a gene comprising a nucleotide sequence of SEQ ID NO: 2.

3. The method of claim 1, wherein the microorganism of the genus *Corynebacterium* is *Corynebacterium glutamicum.*

4. The method of claim 1, further comprising the step of recovering L-citrulline from the cultured microorganism, a culture of the microorganism, a fermentation product of the microorganism, or the culture medium.

5. A composition for producing L-citrulline, the composition comprising a microorganism of the genus *Corynebacterium* having an L-citrulline-producing ability, wherein the activity of a protein comprising an amino acid sequence of SEQ ID NO: 1 is weakened, as compared to its endogenous activity, a culture of the microorganism, a fermentation product of the microorganism, or a combination of two or more thereof.

6. Use of a microorganism of the genus *Corynebacterium* having an L-citrulline-producing ability, wherein the activity of a protein comprising an amino acid sequence of SEQ ID NO: 1 is weakened, as compared to its endogenous activity, in producing L-citrulline.
